(19) Europäisches Patentamt
European Patent Office
Office européen des brevets

(11) Publication number: **0 261 705**
**A1**

(12) **EUROPEAN PATENT APPLICATION**

(21) Application number: 87201575.5

(22) Date of filing: 19.08.87

(51) Int. Cl.[4]: C07D 233/70, C07D 235/02, C07D 487/04, C07D 405/12, A01N 43/50, A01N 43/52, A01N 43/90, //(C07D487/04,235:00,209:00)

(30) Priority: 25.08.86 US 900468

(43) Date of publication of application: 30.03.88 Bulletin 88/13

(84) Designated Contracting States: AT BE CH DE ES FR GB GR IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Gardner, Gary Martin
814 Edythe Street
Manteca California 95336(US)
Inventor: Andrea, Tariq Arthur
1713 Sanchez Avenue
Escalon California 95320(US)
Inventor: Semple, Joseph Edward
3400 Polaris Street
Modesto California 95350(US)

(74) Representative: Hunter, Keith Roger Ian et al
4 York Road
London SE1 7NA(GB)

(54) **Herbicidal imidazolinyl benzoic acids and derivatives.**

(57) Compounds of the Formulae I, II, III or IV



Xerox Copy Centre

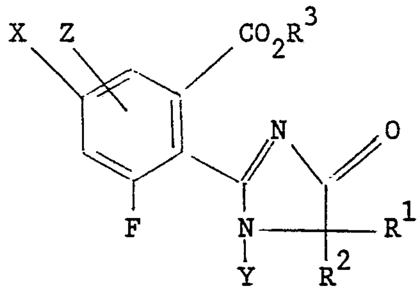

I

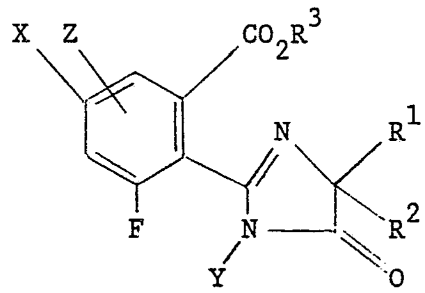

II

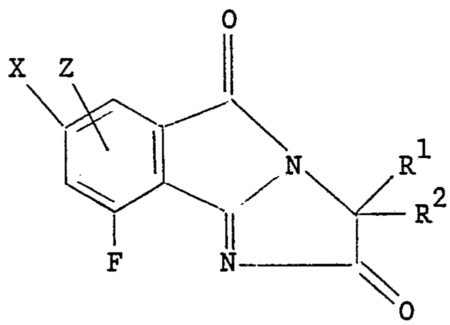

III

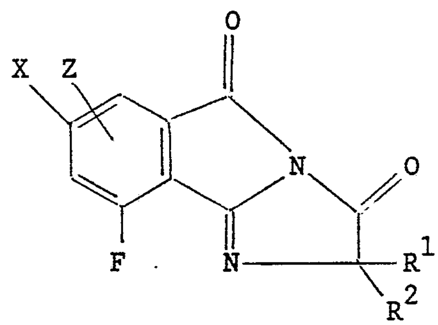

IV

wherein X is hydrogen, $C_1$-$C_3$ alkyl, $C_1$-$C_3$ alkoxy, halogen or nitro;

Y is hydrogen or $C_1$-$C_7$ acyl;

Z is hydrogen, halogen, nitro, CN, $C_1$-$C_3$ alkyl, or $C_1$-$C_3$ haloalkyl; or X and Z when taken together are -CH=CH-CH=CH-;

$R_1$ is $C_1$-$C_4$ alkyl;

$R_2$ is $C_1$-$C_6$ alkyl, $C_3$-$C_6$ cycloalkyl, $C_2$-$C_4$ alkenyl, phenyl, halophenyl or benzyl; or $R_1$ and $R_2$ together with the carbon to which they are attached represent optionally substituted $C_3$-$C_6$ cycloalkyl; and

$R_3$ is hydrogen, optionally substituted $C_1$-$C_{12}$ alkyl, optionally substituted $C_3$-$C_5$ alkenyl or alkynyl, benzyl, cyclohexenylmethyl, ethynylcyclohexyl, ethynylalkyl, pentadienyl, or optionally substituted $C_3$-$C_6$ cycloalkyl, are useful as herbicides and plant growth regulators.

## HERBICIDAL IMIDAZOLINYL BENZOIC ACIDS AND DERIVATIVES

The present invention relates to 3-fluoro-2-(4-oxo-5,5-disubstituted-imidazolin-2-yl)benzoic acids and derivatives thereof, their use as herbicides for controlling undesirable plant growth and to compositions containing such compounds.

U.S. Patent Nos. 4,017,510, 4,067,718 and 4,188,487 describe certain imidazolinyl benzoic acids, esters and salts and derivatives thereof and their use as herbicides. Some of these compounds contain a chlorine atom on the benzoic acid ring. While such compounds do have some herbicidal activity, it is desirable to obtain materials which have a higher level and greater spectrum of activity in both pre-emergence and post-emergence applications.

The present invention is directed to compounds of the Formula I, II, III or IV

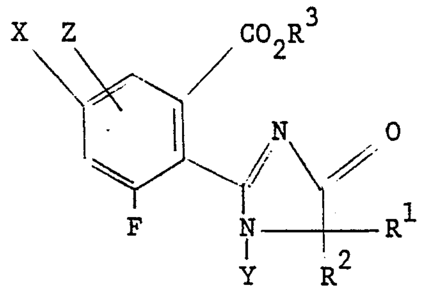

I

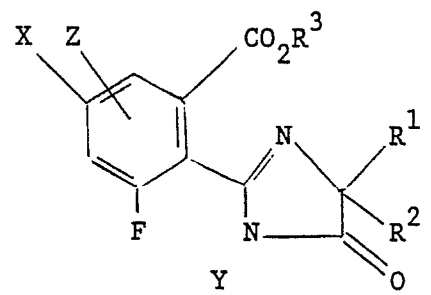

II

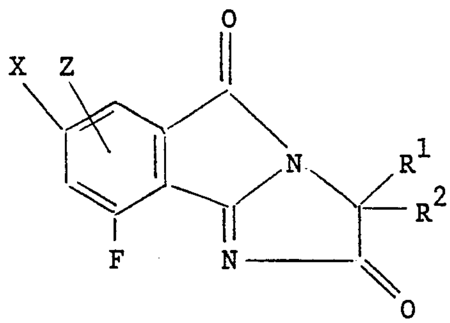

III

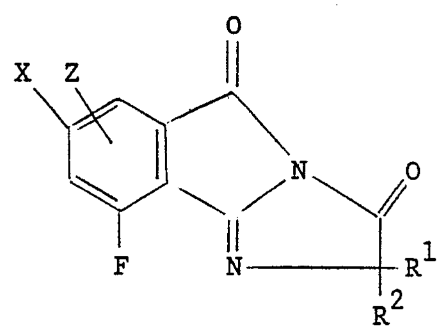

IV

wherein X is hydrogen, $C_1$-$C_3$ alkyl, $C_1$-$C_3$ alkoxy, halogen or nitro; Y is hydrogen or $C_1$-$C_7$ acyl; Z is hydrogen, halogen, nitro, CN, $C_1$-$C_3$ alkyl, or $C_1$-$C_3$ haloalkyl; or X and Z when taken together are -CH=CH-CH=CH-; $R_1$ is $C_1$-$C_4$ alkyl; $R_2$ is $C_1$-$C_6$ alkyl, $C_3$-$C_6$ cycloalkyl, $C_2$-$C_4$ alkenyl, phenyl, halophenyl or benzyl; or when $R_1$ and $R_2$ are taken together with the carbon to which they are attached they may represent $C_3$-$C_6$ cycloalkyl optionally substituted with methyl; except that, for a compound of formula I or II where X is hydrogen, $R^1$ is methyl and $R^2$ is isopropyl, Z is not a 4-methyl substituent; and $R_3$ is hydrogen, $C_1$-$C_{12}$ alkyl optionally substituted with one $C_1$-$C_3$ alkoxy group or one alkoxycarbonyl group or one $C_3$-$C_6$ cycloalkyl group or one phenyl group or one furanyl group or with one to three halogen substituent(s) (preferably chlorine), $C_3$-$C_5$ alkenyl or alkynyl optionally substituted with one or two $C_1$-$C_3$ alkyl group(s) or one phenyl group or with one to two halogen substituent(s) (preferably chlorine), benzyl, cyclohexenylmethyl, ethynylcyclohexyl, ethynylalkyl, pentadienyl or $C_3$-$C_6$ cycloalkyl optionally substituted with one or two $C_1$-$C_3$ alkyl group(s); or a salt forming cation of an alkali metal, ammonia, or an aliphatic amine; and, when $R_1$ and $R_2$ are not the same, the optical isomers and the isomeric mixtures thereof; and, except when $R_3$ is a salt-forming cation, the acid addition salts thereof.

The compounds are useful for controlling undesirable plant species growth at a locus and the invention therefore includes a method of combating plant growth at a locus comprising treating the locus, for example by applying to the foliage of the undesirable plant species or to soil containing seeds, seedlings, or propagating organs of the undesirable plant species, a herbicidally effective amount of an imidazolinyl benzoic acid derivative depicted by Formula I, II, III or IV as defined above. The compounds are also intermediates to each other depending on which of the synthesis routes described below is selected.

Preferred compounds for use as herbicidal agents are those represented by Formula I, II, III or IV above, wherein X is hydrogen, $C_1$-$C_3$ alkyl, $C_1$-$C_3$ alkoxy or halogen; Y is hydrogen or $C_{1-2}$ acyl; Z is hydrogen, halogen or $C_{1-3}$ alkyl; $R_1$ is $C_1$-$C_3$ alkyl; $R_2$ is $C_1$-$C_3$ alkyl or cyclohexyl; or when $R_1$ and $R_2$ are taken together with the carbon to which they are attached they represent cyclohexyl or methylcyclohexyl; $R_3$ is hydrogen, $C_1$-$C_{12}$ alkyl optionally substituted with one $C_1$-$C_3$ alkoxy group or one alkoxycarbonyl group or one $C_3$-$C_6$ cycloalkyl group or one phenyl group or one furanyl group or with one to three halogen substituent(s) (preferably chlorine), $C_3$-$C_5$ alkenyl optionally substituted with one or two $C_1$-$C_3$ alkyl group(s) or one phenyl group or with one or two halogen substituent(s) (preferably chlorine), $C_3$-$C_5$ alkynyl optionally substituted with one or two $C_1$-$C_3$ alkyl group(s) or one phenyl group or with one to two halogen substituent(s) (preferably chlorine), benzyl, cyclohexenylmethyl, ethynylcyclohexyl, ethynylalkyl, pentadienyl or $C_3$-$C_6$ cycloalkyl optionally substituted with one or two $C_1$-$C_3$ alkyl group(s); or a salt forming cation of an alkali metal or ammonia; and, when $R_1$ and $R_2$ are not the same, the optical isomers and the isomeric mixtures thereof; and, except when $R_3$ is a salt-forming cation, the acid addition salts thereof.

In one preferred form of the invention, the compounds are those represented by Formula I, II, III or IV wherein X is hydrogen, methyl, chlorine or fluorine; Y is hydrogen or $C_1C_2$-acyl; Z is hydrogen, fluorine, methyl or ethyl; $R_1$ is $C_1C_3$-alkyl; $R_2$ is $C_1$-$C_3$ alkyl or cyclohexyl; or, when $R_1$ and $R_2$ are taken together with the carbon to which they are attached, they represent cyclohexyl or methylcyclohexyl; $R_3$ is hydrogen, $C_1$-$C_{12}$ alkyl, $C_3$-$C_5$ alkenyl optionally substituted with one or two $C_1$-$C_3$ alkyl substituents, $C_3$-$C_5$ alkynyl optionally substituted with one or two $C_1$-$C_3$ alkyl substituents, benzyl, cyclohexenylmethyl or pentadienyl; and, when $R_1$ and $R_2$ are not the same, the optical isomers and the isomeric mixtures thereof; and the addition salts thereof.

Still more preferred are compounds represented by Formula I, II, III or IV wherein $R_1$ is methyl; $R_2$ is isopropyl; Y is hydrogen; Z is hydrogen, fluorine, methyl or ethyl; and (1) X is hydrogen, methyl, fluorine or chlorine; and $R_3$ is hydrogen, or the alkali metal and ammonium salts thereof; or (2) X is hydrogen, methyl, fluorine or chlorine; $R_3$ is $C_1$-$C_{12}$ alkyl optionally substituted with one $C_1$-$C_3$ alkoxy group or one $C_3$-$C_6$ cycloalkyl group or one phenyl group or one furanyl group or with one to three halogen substituent(s) preferably chlorine. Particularly useful compounds have the Formula I or II wherein X, Y, Z and $R_3$ are hydrogen, and $R_1$ is methyl and $R_2$ is isopropyl.

A further preferred group of compounds in accordance with the invention are those of formulae I, II, III and IV as defined above where Z is hydrogen.

Non-limiting illustrative examples of compounds within the scope of the invention include:
3-fluoro-5-methyl-2-(5-isopropyl-5-methyl-4-oxo-2-imidazolin-2-yl)benzoic acid, methyl ester,
3-fluoro-5-ethyl-2-(5-isopropyl-5-methyl-4-oxo-2-imidazolin-2-yl)benzoic acid, methyl ester,
3-fluoro-5-methyl-2-(5-isopropyl-5-methyl-4-oxo-2-imidazo-lin-2-yl)benzoic acid,
3-fluoro-5-ethyl-2-(5-isopropyl-5-methyl-4-oxo-2-imidazolin-2-yl)benzoic acid,
3,4,5-trifluoro-2-(5-isopropyl-5-methyl-4-oxo-2-imidazolin-2-yl)benzoic acid,
3-fluoro-5-isopropoxy-2-(5-isopropyl-5-methyl-4-oxo-2-imidazolin-2-yl)benzoic acid,
3,5-difluoro-2-(5-isopropyl-5-methyl-4-oxo-2-imidazolin-2-yl)benzoic acid, n-propyl ester,
3-fluoro-4-chloro-2-(5-isopropyl-5-methyl-4-oxo-2-imidazolin-2-yl)benzoic acid, 1,1-dimethylallyl ester,
3-fluoro-2-(5-isopropyl-5-methyl-4-oxo-2-imidazolin-2-yl)benzoic acid, octyl ester
(+)-3-fluoro-2-(5-isopropyl-5-methyl-4-oxo-2-imidazolin-2-yl)benzoic acid, ethyl ester,
(+)-3-fluoro-2-(5-isopropyl-5-methyl-4-oxo-2-imidazolin-2-yl)benzoic acid, 2-chloroallyl ester,
3-fluoro-5-nitro-2-(5-ethyl-5-methyl-4-oxo-2-imidazolin-2-yl)benzoic acid,
(-)-3-fluoro-2-(5-isopropyl-5-methyl-4-oxo-2-imidazolin-2-yl)benzoic acid, 2-propynyl ester,
(-)-3-fluoro-2-(5-isopropyl-5-methyl-4-oxo-2-imidazolin-2-yl)benzoic acid, compound with isopropylamine salt,
3-fluoro-5-ethyl-2-(5-isopropyl-5-methyl-4-oxo-2-imidazolin-2-yl)benzoic acid, sodium salt,
3-fluoro-2-(5-isopropyl-5-methyl-4-oxo-2-imidazolin-2-yl)benzoic acid, 1-methyl-2-butenyl ester;
and the corresponding derivatives of Formula II, III or IV.

The invention also includes a process for the preparation of a compound of Formula I, II, III or IV as defined above comprising carbonating a compound of Formula V, or its tautomer,

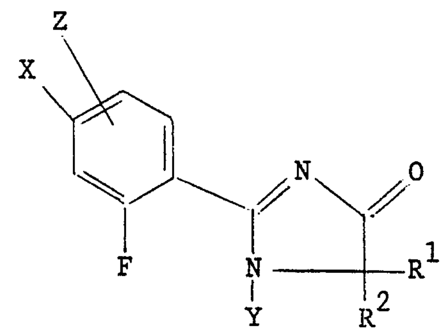

V

wherein $R^1$, $R^2$, X, Y and Z are as defined in claim 1, by treatment with an organo metallic compound and carbon dioxide to yield a compound of Formula I or II where $R^3$ is H; optionally converting the free acid of Formula I or II where $R^3$ is H to the desired ester, salt or acid addition salt of Formula I or II where $R^3$ is other than H; or optionally heating the free acid of Formula I or II where $R^3$ is H in the presence of a condensing agent to give a compound of Formula III or IV.

Alternatively compounds of Formula I, II, III or IV may be prepared by cyclising a compound of Formula VI

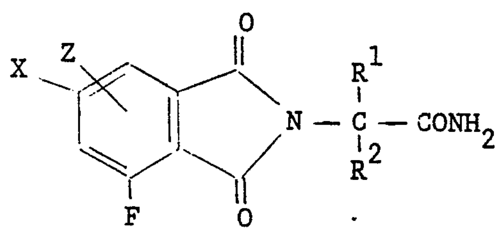

VI

wherein $R^1$, $R^2$, X, Y and Z are as defined in claim 1, to give a compound of Formula III or IV; optionally hydrolysing the compound of Formula III or IV to a compound of Formula I or II; and, where $R^3$ is hydrogen, optionally converting the free acid of Formula I or II to the desired ester, salt or acid addition salt of Formula I or II where $R^3$ is other than H.

The compounds of Formula I or II where $R^3$ is H may be prepared by (1) treating a compound of the Formula V with n-butyl lithium and N,N,N',N'-tetramethylethylenediamine in ether at -78° to -25°C, (2) adding carbon dioxide at -78°C, and (3) treating with aqueous hydrochloric acid.

The compounds of Formulae III and IV may be prepared by heating the acids of Formula I with acetic anhydride. The compounds of Formula I or II where $R^3$ is other than H or a salt may be prepared by treating the compounds of Formulae III and IV with an alcohol in the presence of a 0.1 to 0.5 equivalent of the appropriate alkali metal alkoxide, followed by acetic acid.

The compounds of Formula V or their tautomers may be prepared by treating the appropriate 2-fluorobenzoic acid chloride with an appropriate alpha-amino-alpha,alpha-disubstituted acetamide in the presence of triethylamine in an inert solvent at 0°C to ambient temperature, followed by cyclization of the resultant intermediate bisamide with sodium hydride, DBU (1,8-diazabicyclo[5.4.0]undec-7-ene) or any suitable basic catalyst in a refluxing solvent that forms an azeotrope with water. Suitable solvents include toluene, xylene, and 0-dichlorobenzene.

The compounds of Formula I or II where $R^3$ is H may also be prepared by treating a compound of Formula III or IV with an excess of hydrochloric acid in the presence of a water-miscible solvent and treating the resulting spirolactone with base. Alternatively, one compound of Formula I may be prepared by treating a compound of Formula III or IV with a catalytic to stoichiometric amount of an alkali metal hydroxide in the presence of an aqueous alcoholic solvent and treating the resultant solution with a mineral acid. The acids can be conventionally converted to the corresponding salts.

The compounds of Formulae II and IV may also be prepared by cyclizing the corresponding fluorophthalimidocarboxamide precursors of Formula VI above by pyrolysis, or more conveniently by treatment with sodium hydride, DBU (1,8-diazabicyclo[5.4.0]undec-7-ene), or any suitable basic catalyst in a refluxing solvent that forms an azeotrope with water. Suitable solvents include toluene, xylene, and o-dichlorobenzene.

The compounds of the Formula I may also be prepared by cyclization of the corresponding N-(carbamoylalkyl)phthalamate with an alkali metal hydride or by refluxing with excess aqueous sodium hydroxide optionally followed by treatment with hydrochloric acid.

The above processes are broadly disclosed in U.S. patents 4,017,510, 4,067,718 and 4,188,487.

The compounds of Formula I, II, III or IV have been found to be useful for controlling and combatting undesirable plant growth at a locus by applying an effective amount of the compound to the locus of the plant. The compounds are also selective with respect to some crop species such as corn, rice, wheat, grain sorghum and the like, particularly in post-emergence application.

For application, a compound of Formula I, II, III or IV ordinarily is applied most effectively by formulating it with a suitable inert carrier or surface-active agent, or both. The invention, therefore, also includes compositions comprising an inert carrier or surface-active agent, or both, and as active ingredient at least one compound of Formula I, II, III or IV.

The term "carrier" as used herein means an inert solid or liquid material, which may be inorganic or organic and of synthetic or natural origin, with which the active compound is mixed or formulated to facilitate its application to the plant, seed, soil or other object to be treated, or its storage, transport and/or handling. Any of the materials customarily employed in formulating pesticides, herbicides, or fungicides -i.e., horticulturally acceptable carriers --are suitable.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements such as, for example, carbon and sulphur; natural and synthetic resins such as, for example, coumarone resins, polyvinyl chloride, and styrene polymers and copolymers; bitumen; waxes such as, for example, beeswax, paraffin wax, and chlorinated mineral waxes; solid fertilizers, for example, superphosphates; and ground, naturally-occurring, fibrous materials, such as ground corncobs.

Example of suitable liquid carriers are water, alcohols such as isopropyl alcohol and glycols; ketones such as acetone, methyl ethyl ketone, methyl isobutyl ketone and cyclohexanone; ethers such as cellosolves; aromatic hydrocarbons such as benzene, toluene and xylene; petroleum fractions such as kerosene and light mineral oils; chlorinated hydrocarbons such as carbon tetrachloride, perchloroethylene and trichloromethane. Also suitable are liquefied, normally vaporous and gaseous compounds. Mixtures of different liquids are often suitable.

The surface-active agent may be an emulsifying agent or a dispersing agent or a wetting agent; it may be nonionic or ionic. Any of the surface-active agents usually applied in formulating herbicides or insecticides may be used. Examples of suitable surface-active agents are the sodium and calcium salts of polyacrylic acids and lignin sulfonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example, p-octylphenol or p-octylcresol, with ethylene oxide and/or propylene oxide; sulfates or sulfonates of these condensation products, alkali or alkaline earth metal salts, preferably sodium salts, of sulfuric or sulfonic acid esters containing at least 10 carbon atoms in the molecule, for example, sodium lauryl sulfate, sodium secondary alkyl sulfates, sodium salts of sulfonated castor oil, and sodium alkylaryl sulfonates such as sodium dodecylbenzene sulfonate; and polymers of ethylene oxide and copolymers of ethylene oxide and propylene oxides.

The compositions of the invention may be prepared as wettable powders, dusts, granules, solutions, emulsifiable concentrates, emulsions, suspension concentrates and aerosols. Wettable powders are usually compounded to contain 25 to 75% by weight of active compound and usually contain, in addition to the solid carrier, 3-10% by weight of a dispersing agent, 2-15% of a surface-active agent and, where necessary, 0-10% by weight of stabilizer(s) and/or other additives such as penetrants or stickers. Dusts are usually formulated as a dust concentrate having a similar composition to that of a wettable powder but without a dispersant or surface-active agent, and are diluted in the field with further solid carrier to give a composition usually containing 0.5-10% by weight of the active compound. Granules are usually prepared to have a size between 10 and 100 BS mesh (1.676-0.152 mm), and may be manufactured by agglomeration or impregnation techniques. Generally, granules will contain 0.5-25% by weight of the active compound, 0-1% by weight of additives such as stabilizers, slow release modifiers and binding agents. Emulsifiable concentrates usually contain, in addition to the solvent and, when necessary, cosolvent, 10-50% weight per volume of the active compound, 2-20% weight per volume emulsifiers and 0-20% weight per volume of appropriate additives such as stabilizers, penetrants and corrosion inhibitors. Suspension concentrates are

compounded so as to obtain a stable, non-sedimenting, flowable product and usually contain 10-75% weight of the active compound, 0.5-5% weight of dispersing agents, 1-5% of surface-active agent, 0.1-10% weight of suspending agents, such as defoamers, corrosion inhibitors, stabilizers, penetrants and stickers, and as carrier, water or an organic liquid in which the active compound is substantially insoluble; certain organic solids or inorganic salts may be dissolved in the carrier to assist in preventing sedimentation or as antifreeze agents for water.

Of particular interest in current practice are water-dispersible granular formulations. There are in the form of dry, hard granules that are essentially dust-free, and are resistant to attrition on handling, thus minimizing the formation of dust. On contact with water, the granules readily disintegrate to form stable suspensions of the particles of active material. Such formulations contain 90% or (up to 95%) more by weight of finely divided active material, 3-7% by weight of a blend of surfactants, which act as wetting, dispersing, suspending and binding agents, and may contain up to 3% by weight of a finely divided carrier, which acts as a resuspending agent.

Aqueous dispersions and emulsions, for example, compositions obtained by diluting a wettable powder or a concentrate according to the invention with water, also lie within the scope of the present invention. The said emulsions may be of the water-in-oil or of the oil-in-water type, and may have thick, mayonnaise-like consistency.

It is evident from the foregoing that this invention contemplates compositions containing as little as about 0.5% by weight to as much as about 95% by weight of a compound of Formula I, II, II or IV as the active ingredient.

The compositions of the invention may also contain other ingredients, for example, other compounds possessing pesticidal, especially insecticidal, acaricidal, herbicidal or fungicidal properties, as are appropriate to the intended purpose.

Protection of a locus or area from undesirable plants is effected by applying a compound of Formula I, II, III or IV, ordinarily in a composition of one of the aforementioned types, to soil in which the seeds of the unwanted plants are present, or to the foliage of the unwanted plants. The active compound, of course, is applied in an amount sufficient to exert the desired action.

The amount of the compound of the invention to be used in combatting undesired plants will naturally depend on the condition of the plants, the degree of activity desired, the formulation used, the mode of application, the climate, the season of the year, and other variables. Recommendations as to precise amounts are, therefore, not possible. In general, however, application to the locus to be protected of from 0.1 to 10.0 kg per hectare of the compound of Formula I, II, III or IV will be satisfactory.

The invention is illustrated by the following examples which are presented for the purpose of illustration, and should not be regarded as limiting the invention in any way. The identity of the products was confirmed by elemental, infrared and nuclear magnetic resonance spectral (NMR) analysis as necessary.

Example 1 -3-Fluoro-2-(5-isopropyl-5-methyl-4-oxo-2-imidazolin-2-yl)benzoic acid

To a cooled (-78°C) solution of 2.34 g of 2-(2-fluorophenyl)-5-methyl-5-isopropyl-1,3-imidazolin-4-one and 3.02 g N,N,N',N'-tetramethylethylenediamine in 150 ml of dry ether was added 8.2 ml of 2.7 M n-butyllithium in hexane over 10 minutes. After 30 minutes at -78°C, the haze orange solution was warmed to -20 to -30°C for 2.5 hours and recooled to -78°C with the addition of excess carbon dioxide. After 10 minutes, the solution was warmed to room temperature followed by addition of 75 ml of water and 6N hydrochloric acid to give a pH of 7. The ether was decanted, and the aqueous phase was brought to pH 10 with 10% sodium hydroxide. The resulting aqueous solution was extracted thrice with 75 ml of ethyl acetate, concentrated, cooled to 0°C, acidified with 6N hydrochloric acid to pH 3, extracted thrice with methylene chloride, adjusted to pH 6, extracted thrice with ethyl acetate, and dried ($MgSO_4$). The yellow crystals from the methylene chloride and ethyl acetate extracts totalled 0.37 g; m.p.: 211-213°C. Concentration of the aqueous solution and ethanol extraction of the residue gave 2.19 g of colorless solid. Trituration of the above with ethyl acetate gave a total of 1.26 g of the desired product, m.p.: 220-222°C.

Example 2 -Cyclized derivatives of 3-fluoro-2-(5-isopropyl-5-methyl-4-oxo-2-imidazolin-2-yl)benzoic acid

A stirred solution of 0.40 g of the acid of Example 1 above in 20 ml of acetic anhydride was refluxed for 1 hour. After removal of acetic anhydride invacuo, 0.37 g of a mixture of the desired products was obtained as an oil that solidified to a light brown solid; m.p. 86-92°C.

Example 3 -Methyl 3-fluoro-2-(5-methyl-4-oxo-2-imidazolin-2-yl)benzoate

To a solution of 190 mg of the material of Example 2 above in 15 ml of dry methanol was added 19.7 mg of sodium methoxide. The mixture was stirred at room temperature for 1 hour, then acidified to pH 5-6 with acetic acid, concentrated, dissolved in ethyl acetate, and chromatographed with ethyl acetate as eluent, and concentrated to give 212.0 mg of the desired product as an oily solid. The product was recrystallized from diethyl ether-hexane to give the desired product as a colorless solid; m.p. 156-158°C.

Example 4 -3-Fluoro-5-methyl-2-(5-isopropyl-5-methyl-4-oxo-2-imidazolin-2-yl)benzoic acid

To a cooled -78°C solution of 0.993 g of 2-(2-fluoro-4-methylphenyl)-5-methyl-5-isopropyl-1,3-imidazolin-4-one and 1.21 g of N,N,N'N'-tetramethylethylenediamine in 60 ml dry ether was added 3.39 ml of 2.6 M n-butyllithium in hexane over 5 minutes while maintaining the temperature at -78 to -70°C. The resulting orange solution was stirred at -78°C for 30 minutes, warmed to -20°C and held at -30 to -20°C for 2 hours 45 minutes. The dark red-brown solution was recooled to -78°C and treated with excess carbon dioxide. Ater stirring for 10 minutes at -78°C, the reaction mixture was warmed to room temperature, the ether was removed, 30 ml of water and sufficient 10% sodium hydroxide were added to give a pH of 10-11. The solution was extracted thrice with ethyl acetate, and the aqueous phase was reduced in volume to 20 ml, cooled in an ice bath and acidified with 6N hydrochloric acid to pH 3. The resulting mixture was stirred at 0°C for 45 minutes and filtered. The solid was washed with cold water and dried to give the desired product as a colorless solid; m.p. 264-266°C.

Examples 5-8

Following procedures similar to those described in Examples 1-4 above, the following 3-fluoro-2-(5-isopropyl-5-methyl-4-oxo-2-imidazolin-2-yl)benzoic acid derivatives were prepared as set forth in Table I.

Table 1

| Example | Formula of Invention | X | $R^3$ | M.P. (°C) |
|---|---|---|---|---|
| 5 | III and IV | $CH_3$ | - | 98-108 |
| 6 | I and II | F | H | 250-251.5 |
| 7 | III and IV | F | - | oil |
| 8 | I and II | F | $CH_3$ | glass |

Example 9 Activity with Respect to Plants

In the following example, the species of plants that were tested were:

| | Abbreviation |
|---|---|
| Barnyardgrass (watergrass) - *Echinochloa crus-galli* | BYGR |
| Downy brome - *Bromus tectorum* | DOBR |
| Yellow foxtail - *Setaria glauca* | YEFT |
| Sicklepod - *Cassia obtusifolia* | SIPO |
| Velvetleaf - *Abutilon theophrasti* | VELE |
| Garden cress - *Lepidium sativum* | GACR |
| Johnsongrass - *Sorghum halepense* | JOGR |
| Morningglory - *Ipomoea* sp. | MOGL |
| Field bindweed - *Convolvulus arvensis* | FIBW |
| Nightshade - *Solanum* sp. | NISH |
| Blackgrass - *Alopecurus myosuroides* | BLGR |
| Yellow millet - *Panicum miliaceum* | YEMI |
| Large crabgrass - *Digitaria sanguinalis* | LACG |
| Redroot pigweed - *Amaranthus retroflexus* | RRPW |
| Hemp sesbania - *Sesbania exaltata* | HESE |
| Prickly sida - *Sida spinosa* | PRSI |

Test Procedures

The preemergence (soil) herbicidal activity of compounds of Formulae I, II, III or IV was evaluated by planting seeds of downy brome, johnsongrass, yellow foxtail, barnyardgrass, yellow millet, blackgrass, hemp sesbania, velvetleaf, morningglory, prickly sida, sicklepod and garden cress in test tubes, nominally measuring 25 × 200 millimeters, filled about three-quarters full of untreated soil, in each case covered on top with about 2.5 cubic centimeters of soil treated with 0.1 milligram of the test compound, to give a dosage of 2.0 pounds of test compound per acre. The seeds were planted on top of the treated soil and covered with about 1.5 cubic centimeters of untreated soil. The planted soil was held under a controlled regimen of temperature, moisture, and light. At 10 days, the amounts of germination and growth in each tube were evaluated on a 0 to 9 scale, the numeric ratings having the following meanings:

| Rating | Meaning |
|---|---|
| 9 | No living tissue |
| 8 | Plant severely damaged and expected to die |
| 7 | Plant badly damaged, but expected to live |
| 6 | Moderate damage, but complete recovery expected |
| 5 | Intermediate damage (probably unacceptable for crop plants) |
| 3-4 | Observable damage |
| 1-2 | Plant slightly affected, possibly by the chemical, possibly due to biological variability |
| 0 | No visible effect |

The postemergence (foliar) herbicidal activity of compounds of Formulae I, II, III or IV was evaluated by spraying 9-day-old large crabgrass plants, 9-day-old pigweed plants, 6-day-old johnsongrass plants, 9-day-old velvetleaf plants, 8-day-old yellow foxtail plants, 9-day-old sicklepod plants, 5-day-old morningglory plants, 5-day-old barnyardgrass plants, 6-day-old yellow millet plants, 9-day-old nightshade plants, 9-day-old prickly sida plants and 7-day-old field bindweed plants to runoff with 2.4 milliliters of a liquid formulation containing 0.5 milligram of the test compound (1.12 kg per hectare; one pound of the test compound per acre). The sprayed plants were held under a controlled regimen of temperature, moisture and light for 7 to 8 days, when the effect of the test compound was evaluated visually, the results being rated on the 0 to 9 scale described above.

Results of the preemergence and postemergence herbicidal activity tests are set forth in Tables I and II.

TABLE I. PREEMERGENCE HERBICIDAL ACTIVITY

| Compound of Example | DOBR | JOGR | YEFT | BYGR | YEMI | BLGR | HESE | VELE | MOGL | PRSI | SIPO | GACR |
|---|---|---|---|---|---|---|---|---|---|---|---|---|
| 1 | 9 | 8 | 9 | 8 | 8 | 9 | 7 | 7 | 7 | 7 | 7 | 7 |
| 2 | 9 | 7 | 9 | 7 | 7 | 8 | 5 | 7 | 6 | 7 | 6 | 8 |
| 3 | 5 | 5 | 4 | 7 | 5 | 9 | 7 | 7 | 5 | 4 | 7 | 8 |
| 4 | 9 | 8 | 9 | 9 | 8 | 7 | 6 | 7 | 7 | 7 | 6 | 7 |
| 5 | 9 | 7 | 9 | 8 | 7 | 8 | 5 | 7 | 7 | 7 | 7 | 8 |
| 6 | 9 | 8 | 9 | 9 | 8 | 7 | 6 | 7 | 7 | 7 | 6 | 7 |
| 7 | 5 | 3 | 6 | 4 | 5 | 5 | 0 | 6 | 7 | 3 | 5 | 7 |
| 8 | 0 | 0 | 0 | 0 | 0 | 7 | 0 | 4 | 7 | 3 | 2 | 7 |

TABLE II. POSTEMERGENCE HERBICIDAL ACTIVITY

| Compound of Example | LACG | JOGR | YEFT | BYGR | YEMI | RRPW | NISH | VELE | MOGL | PRSI | SIPO | FIBW |
|---|---|---|---|---|---|---|---|---|---|---|---|---|
| 1 | 7 | 7 | 7 | 8 | 9 | 7 | 7 | 7 | 7 | 7 | 8 | 9 |
| 2 | 7 | 8 | 8 | 7 | 6 | 8 | 8 | 7 | 7 | 7 | 6 | 7 |
| 3 | 1 | 5 | 1 | 5 | 4 | 7 | 7 | 6 | 7 | 8 | 7 | 7 |
| 4 | 7 | 7 | 8 | 8 | 6 | 8 | 8 | 8 | 8 | 8 | 7 | 8 |
| 5 | 6 | 6 | 7 | 8 | 6 | 8 | 8 | 8 | 8 | 8 | 8 | 8 |
| 6 | 7 | 7 | 8 | 8 | 6 | 8 | 8 | 8 | 8 | 8 | 7 | 8 |
| 7 | 5 | 3 | 6 | 4 | 5 | 5 | 0 | 6 | 7 | 3 | 5 | 7 |
| 8 | 0 | 0 | 0 | 0 | 0 | 5 | 5 | 4 | 8 | 0 | 4 | 6 |

## Claims

1. A compound of the Formula I, II, III or IV

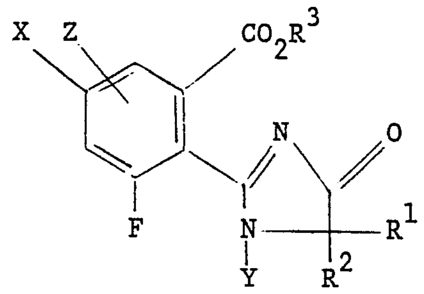

I

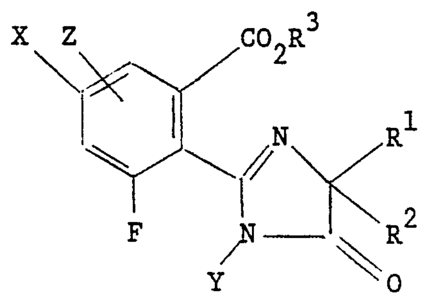

II

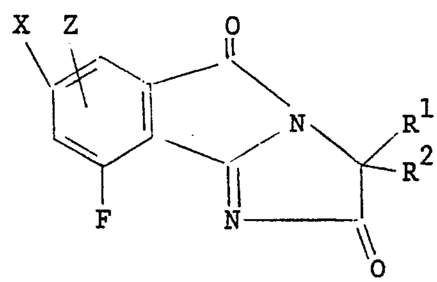

III

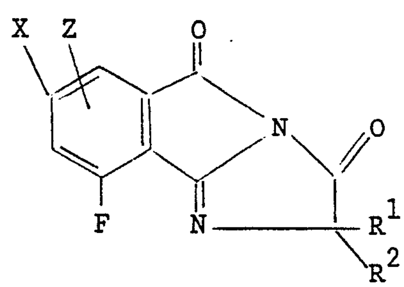

IV

wherein X is hydrogen, $C_1$-$C_3$ alkyl, $C_1$-$C_3$ alkoxy, halogen or nitro; Y is hydrogen or $C_1$-$C_7$ acyl; Z is hydrogen, halogen, nitro, CN, $C_1$-$C_3$ alkyl $C_1$-$C_3$, or $C_1$-$C_3$ haloalkyl; or X and Z when taken together are -CH=CH-CH=CH-; $R_1$ is $C_1$-$C_4$ alkyl; $R_2$ is $C_1$-$C_6$ alkyl, $C_3$-$C_6$ cycloalkyl, $C_2$-$C_4$ alkenyl, phenyl, halophenyl or benzyl; or when $R_1$ and $R_2$ are taken together with the carbon to which they are attached they may represent $C_3$-$C_6$ cycloalkyl optionally substituted with methyl; except that, for a compound of formula I or II where X is hydrogen, $R^1$ is methyl and $R^2$ is isopropyl, Z is not a 4-methyl substituent; and $R_3$ is hydrogen, $C_1$-$C_{12}$ alkyl optionally substituted with one $C_1$-$C_3$ alkoxy group or one alkoxycarbonyl group or on $C_3$-$C_6$ cycloalkyl group or one phenyl group or one furanyl group or with one to three halogen substituent(s), $C_3$-$C_5$ alkenyl or $C_3$-$C_5$ alkynyl optionally substituted with one or two $C_1$-$C_3$ alkyl group(s) or one phenyl group or with one or two halogen substituent(s), benzyl, cyclohexenylmethyl, ethynylcyclohexyl, ethynylalkyl, pentadienyl or $C_3$-$C_6$ cycloalkyl optionally substituted with one or two $C_1$-$C_3$ alkyl group(s); or a salt forming cation of an alkali metal, ammonia, or an aliphatic amine; and, when $R_1$ and $R_2$ are not the same, the optical isomers and the isomeric mixtures thereof; and, except when $R_3$ is a salt-forming cation, the acid addition salts thereof.

2. A compound according to claim 1 wherein X is hydrogen, $C_1$-$C_3$ alkyl, $C_{1-3}$ alkoxy or halogen; Y is hydrogen or $C_{1-2}$acyl; Z is hydrogen, halogen or $C_{1-3}$ alkyl; $R_1$ is $C_1$-$C_3$ alkyl; $R_2$ is $C_1$-$C_3$ alkyl or cyclohexyl; or when $R_1$ and $R_2$ are taken together with the carbon to which they are attached they represent cyclohexyl or methylcyclohexyl; $R_3$ is hydrogen, $C_1$-$C_{12}$ alkyl optionally substituted with one $C_1$-$C_3$ alkoxy group or one alkoxycarbonyl group or one $C_3$-$C_6$ cycloalkyl group or one phenyl group or one furanyl group or with one or three halogen substituent(s), $C_3$-$C_5$ alkenyl optionally substituted with one or two $C_{1-3}$ alkyl group(s) or one phenyl group or with one or two halogen substituent(s), $C_3$-$C_5$ alkynyl optionally substituted with one or two $C_1$-$C_3$ alkyl group(s) or one phenyl group or with one or two halogen substituent(s), benzyl, cyclohexenylmethyl, ethynylcyclohexyl, ethynylalkyl, pentadienyl or $C_3$-$C_6$ cycloalkyl optionally substituted with one or

two $C_1$-$C_3$ alkyl group(s); or a salt forming cation of an alkali metal or ammonia; and, when $R_1$ and $R_2$ are not the same, the optical isomers and the isomeric mixtures thereof; and, except when $R_3$ is a salt-forming cation, the acid addition salts thereof.

3. A compound according to claim 1 or 2, wherein X is hydrogen, methyl, chlorine or fluorine; Y is hydrogen or $C_1$-$C_2$ acyl; Z is hydrogen, fluorine, methyl or ethyl; $R_1$ is $C_1$-$C_3$ alkyl; $R_2$ is $C_1$-$C_3$ alkyl or cyclohexyl; or, when $R_1$ and $R_2$ are taken together with the carbon to which they are attached, they represent cyclohexyl or methylcyclohexyl; $R_3$ is hydrogen, $C_1$-$C_{12}$ alkyl, $C_3$-$C_5$ alkenyl optionally substituted with one or two $C_1$-$C_3$ alkyl substituents, $C_3$-$C_5$ alkynyl optionally substituted with one or two $C_1$-$C_3$ alkyl substituents, benzyl, cyclohexenylmethyl or pentadienyl; and, when $R_1$ and $R_2$ are not the same, the optical isomers thereof, and the isomeric mixtures thereof; and the addition salts thereof.

4. A compound according to claim 1, 2 or 3 wherein $R_1$ is methyl; $R_2$ is isopropyl; Y is hydrogen; Z is hydrogen, fluorine, methyl or ethyl; and (1) X is hydrogen, methyl, fluorine or chlorine; and $R_3$ is hydrogen or the alkali metal and ammonium salts thereof; or (2) X is hydrogen, methyl, fluorine or chlorine; and $R_3$ is $C_1$-$C_{12}$ alkyl optionally substituted with one $C_1$-$C_3$ alkoxy group or one $C_3$-$C_6$ cycloalkyl group or one phenyl group or one furanyl group or with one to three halogen substituent(s).

5. A compound according to any one of claims 1 to 4 wherein X, Y and Z are hydrogen.

6. A compound according to any one of the preceding claims wherein Z is hydrogen.

7. A process for the preparation of a compound of Formula I, II, III or IV as defined in claim 1 comprising carbonating a compound of Formula V, or its tautomer,

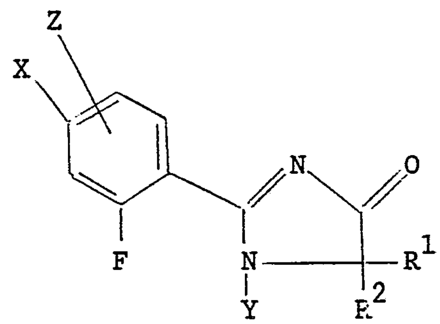

V

wherein $R^1$, $R^2$, X, Y and Z are as defined in claim 1, by treatment with an organo metallic compound and carbon dioxide to yield a compound of Formula I or II where $R^3$ is H; optionally converting the free acid of Formula I or II where $R^3$ is H to the desired ester, salt or acid addition salt of Formula I or II where $R^3$ is other than H; or optionally heating the free acid of Formula I or II where $R^3$ is H in the presence of a condensing agent to give a compound of Formula III or IV.

8. A process for the preparation of Formula I, II, III or IV as defined in claim 1 comprising cyclising a compound of Formula VI

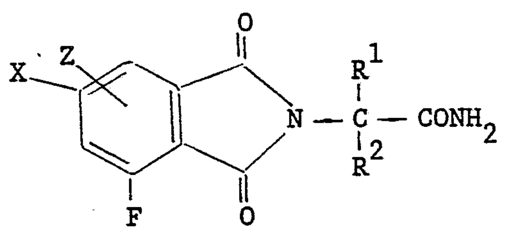

VI

wherein $R^1$, $R^2$, X, Y and Z are as defined in claim 1, to yield a compound of Formula III or IV; optionally hydrolysing the compound of Formula III or IV to a compound of Formula I or II; and, where $R^3$ is hydrogen, optionally converting the free acid of Formula I or II to the desired ester, salt or acid addition salt of Formula I or II where $R^3$ is other than H.

9. A compound of Formula I, II, III or IV when prepared by the process of claim 7 or 8.

10. A herbicidal composition which comprises a compound according to any one of claims 1 to 6 or 9 and at least one surface-active agent and/or carrier.

11. A method of controlling undesirable plant growth at a locus which comprises treating the locus with a compound according to any one of claims 1 to 6 or 9 or a composition according to claim 10.

European Patent Office

# EUROPEAN SEARCH REPORT

Application Number

EP 87 20 1575

## DOCUMENTS CONSIDERED TO BE RELEVANT

| Category | Citation of document with indication, where appropriate, of relevant passages | Relevant to claim | CLASSIFICATION OF THE APPLICATION (Int. Cl.3) |
|---|---|---|---|
| X | EP-A-0 151 744 (HOECHST AG)<br>* examples 16, 17 in combination with page 14, line 1, page 5, line 7 - page 6, line 27 * | 1-6,9 | C 07 D 233/70<br>C 07 D 235/02<br>C 07 D 487/04<br>C 07 D 405/12<br>A 01 N 43/50<br>A 01 N 43/52<br>A 01 N 43/90 //<br>(C 07 D 487/04<br>C 07 D 235:00<br>C 07 D 209:00 ) |
| Y | | 7,8,10, 11 | |
| Y | EP-A-0 133 311 (AMERICAN CYANAMID CO.)<br>* claims 1, 4, 5, 7, 9, 10; page 8, lines 1-28, page 10; page 17, line 25 - page 18, line 8; page 19 * | 7,8,10, 11 | |
| A | * example 15 * | 1 | |
| Y,D | US-A-4 017 510 (LOS)<br>* column 1, line 20 - column 3, line 17 * | 7,8,10, 11 | |
| A | EP-A-0 135 711 (AMERICAN CYANAMID CO.)<br>* claims 1-4, 6-10; example 4; page 38, compound 4; page 43, compound 6 * | 1,7,8, 10,11 | **TECHNICAL FIELDS SEARCHED (Int. Cl.3)** |
| A | EP-A-0 133 310 (AMERICAN CYANAMID CO.)<br>* claims 1, 2, 7-10; table I, page 114, compound 2; example 27a; page 127; compound 2 * | 1,7,8, 10,11 | C 07 D 233/00<br>C 07 D 235/00<br>C 07 D 487/00<br>C 07 D 405/00<br>A 01 N 43/00 |
| A | GB-A-2 167 062 (SHELL INTERNATIONALE RESEARCH MAATSCHAPPIJ B.V.)<br>* claims 1, 11-13 * | 1,10,11 | |

The present search report has been drawn up for all claims

| Place of search | Date of completion of the search | Examiner |
|---|---|---|
| BERLIN | 27-11-1987 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document